# EUROPEAN PATENT SPECIFICATION

(11) **EP 3 433 265 B1**
(45) Date of publication and mention of the grant of the patent: **05.08.2020**
(21) Application number: 17712505.1
(22) Date of filing: 22.03.2017
(51) Int. Cl.: C07K 14/435, C07K 14/705, C12N 15/12

(54) **PHEROMONAL RECEPTOR OF SPODOPTERA LITTORALIS AND IDENTIFICATION OF NATURAL LIGAND OF SAID RECEPTOR AND USES THEREOF**
PHEROMONREZEPTOR AUS SPODOPTERA LITTORALIS UND IDENTIFIZIERUNG EINES NATÜRLICHEN LIGANDEN DES BESAGTEN REZEPTORS UND VERWENDUNGEN DAVON
RÉCEPTEUR DE PHÉROMONES DE SPODOPTERA LITTORALIS ET IDENTIFICATION DU LIGAND NATUREL DUDIT RÉCEPTEUR ET UTILISATIONS CORRESPONDANTES

(30) Priority: 23.03.2016 EP 16305329
(43) Date of publication of application: 30.01.2019
(73) Proprietor: Institut national de recherche pour l'agriculture, l'alimentation et l'environnement, 75007 Paris (FR); Sorbonne Université, 75006 Paris (FR); Centre National de la Recherche Scientifique (CNRS), 75016 Paris (FR)
(72) Inventor: JACQUIN-JOLY, Emmanuelle, 78000 Versailles (FR); DE FOUCHIER, Arthur, 78000 Versailles (FR); MONTAGNE, Nicolas, 93110 Rosny-sous-Bois (FR)
(74) Representative: Regimbeau
(86) International application number: PCT/EP2017/056828
(87) International publication number: WO 2017/162739

(56) References cited:
- WO-A2-2013/034666
- EMMANUELLE JACQUIN-JOLY ET AL: "Candidate chemosensory Genes in Female Antennae of the Noctuid Moth Spodoptera littoralis", INTERNATIONAL JOURNAL OF BIOLOGICAL SCIENCES, vol. 8, no. 7, 1 January 2012 (2012-01-01) , pages 1036-1050, XP055299425, Australia ISSN: 1449-2288, DOI: 10.7150/ijbs.4469 -& Emmaneulle Jacquin-Joly ET AL: "Candidate chemosensory Genes in Female Antennae of the Noctuid Moth Spodoptera littoralis - supplementary material", International Journal of Biological Sciences, 1 January 2012 (2012-01-01), pages 1-21, XP055299427, Retrieved from the Internet: URL:http://www.ijbs.com/v08/p1036/ijbsv08p 1036s1.pdf [retrieved on 2016-09-02]
- KOUTROUMPA FOTINI A ET AL: "Sex in the night: Fatty acid-derived sex pheromones and corresponding membrane pheromone receptors in insects", BIOCHIMIE, MASSON, PARIS, FR, vol. 107, 24 July 2014 (2014-07-24), pages 15-21, XP029105019, ISSN: 0300-9084, DOI: 10.1016/J.BIOCHI.2014.07.018

### SEQUENCE LISTING

<110> INSTITUT NATIONAL DE LA RECHERCHE AGRONOMIQUE
   UNIVERSITE PIERRE ET MARIE CURIE (PARIS 6)
   CENTRE NATIONAL DE LA RECHERCHE SCIENTIFIQUE (CNRS)
<120> NOVEL PHEROMONAL RECEPTOR of Spodoptera littoralis (Lepidoptera, Noctuidae) AND IDENTIFICATION OF NATURAL LIGAND OF SAID RECEPTOR AND USES THEREOF
<130> B372081D34737
<150> EP16305329.1
   <151> 2016-03-23
<160> 4
<170> PatentIn version 3.5
<210> 1
   <211> 397
   <212> PRT
   <213> Spodoptera littoralis
<400> 1
   Met Thr Asn Gln Lys Asp Leu Asp Phe Glu Asn Ile Phe Lys Ile Thr
   1               5                   10                  15
   Thr Thr Ala Leu His Ile Ser Gly Ser His Pro Ser Val Pro Lys Asp
               20                  25                  30
   Leu Lys Trp Ala Leu Lys Phe Thr Ile Leu His Gly Thr Phe Ala Leu
           35                  40                  45
   Gly Phe Val Thr Val Ile Tyr Ser Ile Ile Tyr His Asp Leu Lys Glu
       50                  55                  60
   Gln Asn Phe Val Gln Ile Cys Lys Asp Cys Val Val Phe Val Leu Phe
   65                  70                  75                  80
   Cys Val Thr Ser Leu Gln Tyr Cys Val Leu Leu Ile His Gln Asp Asn
                   85                  90                  95
   Leu Val Leu Leu Ile Lys Asn Ile Asn Ser Asp Tyr Glu Gln Met Gln
               100                 105                 110
   Asn Leu Ser Asp Lys Glu Lys Gln Leu Met Asp Lys Tyr Met Asn Leu
           115                 120                 125
   Gly Ala Lys Val Cys Arg His Trp Phe Val Leu Val Leu Val Thr Cys
       130                 135                 140
   Leu Ile Phe Leu Ile Lys Ser Val Gly Phe Met Cys Tyr Tyr Tyr Leu
   145                 150                 155                 160
   Ile Asn Asp Ile Lys Tyr Ile Pro Tyr Tyr Asp Ile Lys Tyr Pro Glu
                   165                 170                 175
   Phe Ile Glu Glu Arg Lys Asn Glu Asn Phe Ser Val Phe Leu Ile Thr
               180                 185                 190
   Tyr Cys Leu Met Phe Tyr Phe Ala Leu Tyr Ser Val Leu Ile Tyr Val
           195                 200                 205
   Ala Tyr Val Pro Leu Gly Pro Val Phe Met Leu His Ala Ser Gly Leu
       210                 215                 220
   Leu Glu Val Val Lys Asn Arg Ile Asp Asp Leu Phe Ser Asp Ser Asn
   225                 230                 235                 240
   Pro Glu Lys Ile Arg Glu Lys Leu Lys Asp Val Val Met Lys Leu Gln
                   245                 250                 255
   Tyr Ile Tyr Ser Ile Val Asp Asp Met Lys Thr Ile Phe Arg Phe Gly
               260                 265                 270
   Tyr Glu Val Ser Leu Lys Gly Thr Ala Val Leu Leu Pro Val Thr Phe
           275                 280                 285
   Tyr Ala Val Leu Glu Ala Ala Lys Asn Gly Glu Ile Ser Met Glu Phe
       290                 295                 300
   Ile Ser Phe Ile Ala Gly Gly Ile Val Ile Ser Ala Val Pro Cys Tyr
   305                 310                 315                 320
   Tyr Ser Asp Leu Leu Met Glu Lys Gly Glu Ala Val Arg Leu Ser Leu
                   325                 330                 335
   Tyr Met Cys Gly Trp Glu Gln His Tyr Asp Arg Arg Thr Arg Thr Thr
               340                 345                 350
   Leu Gln Leu Met Leu Leu Arg Ala Leu Arg Pro Ile Ala Ile Gln Thr
           355                 360                 365
   Leu Phe Arg Thr Leu Cys Leu Asp Ala Leu Thr Asp Leu Phe Gln Gln
       370                 375                 380
   Ser Tyr Gly Ile Phe Asn Leu Met Asn Ala Met Trp Ser
   385                 390                 395
<210> 2
   <211> 1194
   <212> DNA
   <213> Spodoptera littoralis
<400> 2
   atgacgaacc aaaaggactt ggactttgaa aacattttca aaattacaac gacggcattg      60
   catatcagtg ggtctcatcc cagcgtaccc aaagatttaa aatgggccct aaaatttaca     120
   atactccacg ggacatttgc tttaggattc gtaacagtta tatacagcat catctaccac     180
   gatttaaaag aacaaaactt cgttcaaatt tgcaaagact gcgtagtatt tgttttattc     240
   tgtgtgacat cactccagta ctgcgtctta ctgatccatc aggataacct cgtgttattg     300
   attaaaaata taaattctga ctatgagcag atgcagaact tgtctgataa ggaaaagcag     360
   ttgatggaca aatatatgaa tctaggtgca aaagtttgcc ggcattggtt cgttctggtt     420
   ttagtaactt gcttgatatt ccttataaaa agtgtgggat ttatgtgcta ttactattta     480
   attaatgata tcaaatatat tccctattac gatattaagt acccagagtt cattgaagaa     540
   cggaagaatg aaaatttctc tgtgtttttg attacttatt gtttaatgtt ttactttgcc     600
   ctttattcgg ttttgattta cgttgcgtac gtgccactgg gtccagtatt tatgttgcat     660
   gcaagtggac tacttgaggt ggtcaagaac cggattgacg atctgttttc ggattctaat     720
   cctgaaaaga ttcgggagaa actgaaggac gttgttatga agctgcagta catttacagc     780
   atcgttgatg atatgaagac aatatttagg tttgggtacg aagtgtctct gaaaggtaca     840
   gctgtactac tgcctgtcac cttttatgca gtgcttgagg ctgccaaaaa tggagaaata     900
   agcatggagt tcataagttt catagcagga gggatagtga taagtgcggt tccgtgctac     960
   tacagcgact tgcttatgga gaagggtgaa gctgtacgct tgtccttgta catgtgtggg    1020
   tgggagcagc attacgatcg gcgcacgcgc accacgctgc agttgatgct gctacgggca    1080
   ctcaggccta ttgctataca gactttattc agaactttat gcttggatgc cctaactgac    1140
   ttgtttcaac agtcttatgg tatcttcaac ctgatgaatg ccatgtggag ttaa          1194
<210> 3
   <211> 41
   <212> DNA
   <213> Artificial Sequence
<220>
   <223> forward primer
<400> 3
   aacagatctg cggccgccag acgaagcaga caccaattct a 41
<210> 4
   <211> 41
   <212> DNA
   <213> Artificial Sequence
<220>
   <223> reverse primer
<400> 4
   cctcgagccg cggccgcact catacgttca tacactgcaa g 41

## Description

### FIELD OF THE INVENTION

The invention relates to the identification of the natural ligand of the olfactory/pheromonal receptor SlitOR5 of *Spodoptera littoralis* (Lepidoptera, Noctuidae) and uses thereof.

### BACKGROUND OF THE INVENTION

The ecological interactions between insects and between insects and plants are to a great extent regulated by the emission of volatile organic compounds. The olfactory perception of these airborne cues drives vital behaviors of phytophagous insects, like nectar-feeding, mate and host plant selection, and oviposition. Olfaction is thus at the core of some pesticide-free strategies to control phytophagous crop pests which are responsible for the loss of 1/6 of the world's agricultural production every year.

Insects detect odorants through olfactory sensory neurons (OSNs) housed within sensory hairs - the olfactory sensilla - that cover the surface of antennae and maxillary palps. The watershed event that allowed deciphering of the molecular basis of odorant detection in insects was the discovery of olfactory receptor (OR) genes in the model *Drosophila melanogaster,* which encode transmembrane proteins that bind chemical stimuli at the surface of OSNs and allow for signal transduction.

The insect olfactory receptor can be described as GPCR-like transmembrane receptors, but with an inverted topology compared to classical GPCR (N-terminus of the protein inside the cell, C-terminal outside the cell). They usually have seven transmembrane domains, although predictions suggest some have between 6 and 8 transmembrane domains. Upon binding of a ligand to an extra-cellular portion and/or fragment(s) of the receptor, an electrical signal is sent to the central nervous system, where its integration results in a change in the animal physiology and/or behaviour.

These ORs are encoded by a large gene family. Whereas in *Drosophila melanogaster* 62 different OR genes have been identified, 259 such genes have been identified in the red flour beetle genome *Tribolium castaneum.* Each olfactory neuron is thought to express only one type of OR, forming therefore the cellular basis of odorant discrimination by olfactory neurons. They are synthesized in the endoplasmatic reticulum, transported and eventually concentrated at the cell surface membrane of the sensilla neurone.

Each OSN usually expresses a highly conserved co-receptor named ORco together with one OR that is responsible for the odor response spectrum of the OSN.

Insect ORs, along with ORco and effectors (transmembrane proteins, intracellular enzymes and channels, possibly modulated by G-proteins), are the components of a modular signaling system that connects the extra-cellular inputs to intra-cellular electrical responses and associated behaviours.

Another family of chemosensory receptors, named variant ionotropic receptors (IRs), has been characterized more recently. Compared to ORs, IRs are expressed in a smaller population of OSNs and are tuned to complementary chemical classes of odorants.

The olfactory capacities of an insect thus depend to a great extent on the repertoire of OR genes expressed and on the functional properties of OR proteins, notably their sensitivity and the breadth of their response spectrum. Until now, large OR repertoires have been investigated only in *D. melanogaster* and in the primary malaria vector mosquito *Anopheles gambiae.* These pioneering works notably demonstrated that ORs are involved in the detection of multiple chemical families (including alcohols, esters, aromatics and terpenes) and that, when stimulated with high doses of odorants, ORs exhibit a continuum of tuning breadths from narrowly to broadly tuned.

In phytophagous insects, only a few OR-ligand couples have been identified apart from moth sex pheromone receptors.

Olfactory receptor genes and gene products in insects can modulate various physiological processes and are potential causative agents of many behaviors. The insect olfactory receptors are of critical importance to both the peripheral and central olfactory response within the nervous system, leading to physiological/behavioral response.

Known and uncharacterized insect olfactory receptors currently constitute major targets for the research of chemicals for the control of insect populations. For some insect olfactory receptors, a possible pheromone-binding role has been assigned. There are ongoing efforts to identify new olfactory and pheromonal receptors which can be used to screen for new agonists and antagonists having potential applications in the development of strategies adapted to the control of insect populations.

The sense of smell allows chemical communications between living organisms (invertebrates to mammals) and with their environment. Perception and discrimination of thousands of odorants is made through olfaction. Such chemical signaling may modulate behavior of most animal species which rely on odorant compounds to identify kin or mate, to locate food or to recognize territory for instance.

The (Z,E)-9,11-tetradecadienyl acetate (Z9,E11-14:OAc) has been described as a component of the *Spodoptera littoralis* sex pheromonal bouquet as soon as the early seventies (see pherobase for a reference: www.pherobase.com/).

The noctuid moth *S. littoralis* is a highly polyphagous species whose larvae are pests of more than 80 crops in Africa, the South-West Asia and the Mediterranean basin. *S. littoralis* has been established as a model in the fields of chemical ecology and neurobiology of olfaction, and several behaviorally active odorants (e.g. sex pheromone components and host plant volatiles) and their corresponding OSNs have notably been identified. Its antennal transcriptome was one of the first to be sequenced and 35 candidate ORs were initially identified. Jacquin-Joly et al. (Int. J. Biol Sci. (2012) 8:1036-1050) disclose the identification of 36 olfactory receptors expressed in the antennae of *S. littoralis,* including SlitOR5.

The inventors expressed SlitOR5 of *S. littoralis*: in neurons housed in at1 sensilla of *Drosophila melanogaster.* They characterized the responses of *S. littoralis* SlitOR5 to a variety of odorant molecules previously demonstrated to be physiologically or behaviorally active in *S. littoralis* as well as pheromone components related to Z9,E11-14:OAc and demonstrated that SlitOR5 responds specifically to one odorant.

### SUMMARY OF THE INVENTION

The invention provides a method of identifying an agonist of the signaling activity of an isolated polypeptide which comprises an amino acid sequence having more than 60%, preferably more than 80%, identity with the amino acid sequence of SlitOR5 from *Spodoptera littoralis* defined in SEQ ID NO:1, said method comprising:
a) contacting said SlitOR5 polypeptide with a candidate agent or a sample containing a candidate agent;
b) measuring a signaling activity of said SlitOR5 polypeptide in the presence of said agent or sample; and
c) comparing said activity measured in the presence of said agent or sample to said activity measured in a reaction in which said SlitOR5 polypeptide is contacted with Z9,E11-14:OAc.

The invention encompasses a method of identifying an antagonist or inverse agonist of the signaling activity of an isolated polypeptide which comprises an amino acid sequence having more than 60%, preferably more than 80%, identity with the amino acid sequence of SlitOR5 from *Spodoptera littoralis* defined in SEQ ID NO:1, comprising :
a) contacting said SlitOR5 polypeptide with Z9,E1 1-14:OAc in the presence and in the absence of a candidate agent or a sample containing a candidate agent under conditions permitting the binding of said Z9,E1 1-14:OAc to said SlitOR5 polypeptide; and
b) measuring the binding of said SlitOR5 polypeptide to said Z9,E1 1-14:OAc or a signaling activity of said SlitOR5 polypeptide,
c) comparing the binding or the signaling activity in the presence of said candidate agent or sample with the binding, respectively the signaling activity, in the absence of said agent or sample

In one embodiment of any of the preceding methods, the contacting is performed in or on a cell expressing said SlitOR5 polypeptide. According to the present invention, said cell may be, but is not limited to, Human embryonic kidney cells (Hek293) and derived strains, Human cervical cancer cells (HeLa) and derived strains, Chinese hamster cells (CHO), Monkey cells (COS), primary olfactory cells, Xenopus eggs, insect cells (Sf9, S2, HiFive), yeast or bacteria.

In another embodiment of any of the preceding methods, the contacting is performed in or on synthetic liposomes or virus-induced budding membranes containing a SlitOR5 polypeptide.

In another embodiment of any of the preceding methods, the method is performed using a membrane fraction from cells expressing said SlitOR5 polypeptide.

In a preferred embodiment of either of the preceding methods, the method is performed on a protein chip. In another preferred embodiment of either of the preceding methods, the measuring is performed using a method selected from label displacement, surface plasmon resonance, fluorescence resonance energy transfer, fluorescence quenching, and fluorescence polarization.

According to the present invention, when a functional assay is used, the step of measuring a signalling activity of the SlitOR5 polypeptide may comprise measuring the ion currents through the membranes (electrophysiology, e.g. voltage clamp), OSN depolarization (electroantennography or single sensillum recording) or detecting a change in the level of a second messenger. In another embodiment, the step of measuring a signalling activity comprises measurement of guanine nucleotide binding/coupling or exchange, adenylate cyclase activity, cAMP, Protein Kinase C activity, Protein Kinase A activity phosphatidylinosotol breakdown, diacylglycerol, inositol triphosphate, intracellular calcium, calcium flux, arachinoid acid, MAP kinase activity, tyrosine kinase activity, melanophore assay, receptor initialization assay, or reporter gene expression. When the G-protein binding/coupling or exchange is measured, of all Goc subunits possible preferably the behaviour of GTP-binding protein G protein alpha-q subunit (olfactory), also G-q, is studied.

In a preferred embodiment, the measuring of the signalling activity comprises using a fluorescence or luminescence assay. Fluorescence and luminescence assays may comprise the use of Ca2+ sensitive fluorophores including Fluo-3, Fluo-4 or Fura, (Molecular probes); Ca3-kit family (Molecular Device) and aequorin. Furthermore, said assays may apply an automated fluorometric or luminescent reader such as FDSS (Hammamatsu) or FLIPR (Molecular Device).

In another embodiment, the step of measuring activation of the receptor comprises electrophysiological measurements (known as electroantennography or single sensillum recording; methods well known of persons skilled in the art) performed on wild-type or genetically engineered individuals, expressing a SlitOR5 polypeptide or one related proteins (displaying identity with SlitOR5 of at least 60%).

In another embodiment of any of the preceding methods, the method is a high throughput screening method.

In another embodiment of any of the preceding methods, the candidate agent is part of a chemical library or plant or animal organ extracts. Said animal organ extracts may be, but are not limited to, extracts prepared from male or female bodies, or body parts (e.g. extracted glands, metasoma section, etc.).

In all the above mentioned methods of the present invention an agonist, antagonist, or inverse agonist for a SlitOR5 polypeptide may be used, referred at, applied, or incorporated which binds specifically to and activates a signaling activity of a SlitOR5 polypeptide defined in SEQ ID NO:1.

In addition, in all the above mentioned methods of the present invention, the SlitOR5 polypeptide may have at least 60% identity or higher identity, such as 65, 70, 75, 80, 85, 90, 95 or even 100% to the polypeptide represented in SEQ ID NO:1 but with comparable activity to said SlitOR5 polypeptide. A skilled person knows how to evaluate said activity depending on the assay used.

In addition, in all the above mentioned methods of the present invention, the SlitOR5 polypeptide may be a chimera or an active fragment thereof.

### DEFINITIONS

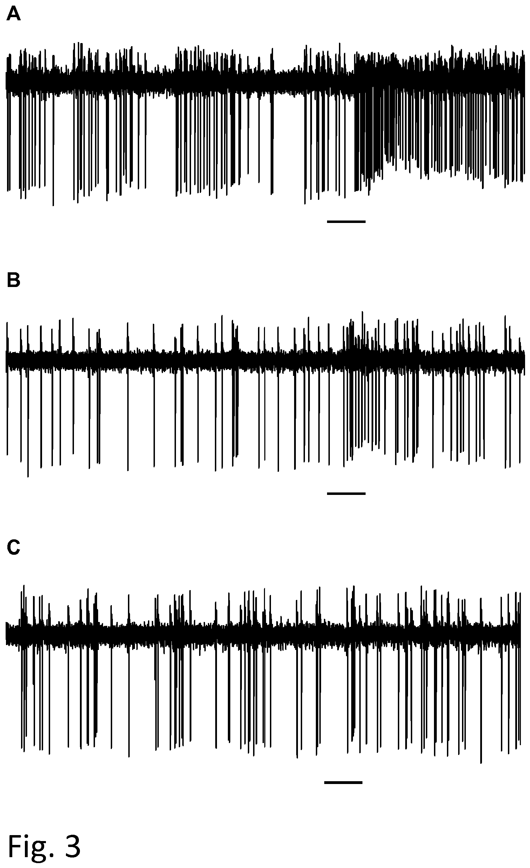

As used herein, the term "SlitOR5 polypeptide" refers to a polypeptide having at least 60% amino acid identity, and preferably 80%, 90%, 95% or higher, including 100% amino acid identity, to the sequence represented in SEQ ID NO:1;
Preferably, said SlitOR5 polypeptide has SlitOR5 activity, i.e., the polypeptide binds Z9,E11-14:OAc.
Optimally, a "SlitOR5 polypeptide" also has SlitOR5 signaling activity as defined herein.
As used herein, the term "SlitOR5 polynucleotide" refers to a polynucleotide that encodes a SlitOR5 polypeptide as defined herein.
"Identity" is defined as the percentage of residues in the amino acid sequence variant that are identical after aligning the sequences and introducing gaps, if necessary, to achieve the maximum percent identity. Methods and computer programs for the alignment are well known in the art. One such computer program is "Align 2", authored by Genentech, Inc., which was filed with user documentation in the United States Copyright Office, Washington, DC 20559, on December 10, 1991.
As used herein, the term "SlitOR5 signaling activity" refers to the initiation or propagation of signaling by a SlitOR5 polypeptide. SlitOR5 signaling activity is monitored by measuring a detectable step in ion currents through the membranes (e.g. voltage clamp), OSN depolarization (electroantennography or single sensillum recording) or a signaling cascade by assaying one or more of the following: stimulation of GDP for GTP exchange on a G protein; alteration of adenylate cyclase activity; protein kinase C modulation; protein kinase A modulation; phosphatidylinositol breakdown (generating second messengers diacylglycerol, and inositol triphosphate); intracellular calcium flux; activation of MAP kinases; modulation of tyrosine kinases; internalization assay; modulation of gene or reporter gene activity; or melanophore assay. A detectable step in a signaling cascade is considered initiated or mediated if the measurable activity is altered by 10% or more above or below a baseline established in the substantial absence of Z9,E11-14:OAc relative to any of the SlitOR5 activity assays described herein below. The measurable activity can be measured directly, as in, for example, measurement of cAMP or diacylglycerol levels. Alternatively, the measurable activity can be measured indirectly, as in, for example, a reporter gene assay. For most of these assays, kits are available in the market. As used herein, the term "detectable step" refers to a step that can be measured, either directly, e.g., by measurement of ion currents through the membranes (e.g. voltage clamp), OSN depolarization (electroantennography or single sensillum recording) or a second messenger (usually an increase in the concentration of the concentration of the intracellular Ca++) or detection of a modified (e.g., phosphorylated) protein, or indirectly, e.g., by monitoring a downstream effect of that step. For example, adenylate cyclase activation results in the generation of cAMP. The increase in the concentration of the concentration of the intracellular Ca++ can be measured directly, e.g., by an assay that uses fluorescent probes such as Fluo4 (e.g.).
As used herein, the term "isolated" refers to a population of molecules (e.g., polypeptides or polynucleotides) the composition of which is less than 50% (by weight), preferably less than 40% and most preferably 2% or less, in contaminating molecules of an unlike nature. When the term "isolated" is applied to a SlitOR5 polypeptide, it is specifically meant to encompass a SlitOR5 polypeptide that is associated with or embedded in a lipid membrane. As used herein, the term Z9,E11-14:OAc refers to a chemical molecule known by a skilled chemist.
As used herein, the term "candidate agent" refers to a molecule to be evaluated in the methods according to the invention. Candidate agents can be natural or synthetic compounds, including, for example, small molecules, compounds contained in extracts of animal, plant, bacterial or fungal cells, as well as conditioned medium from such cells. Candidate agents can be screened from large libraries of synthetic or natural compounds. Numerous means are currently used for random and directed synthesis of various kinds of compounds. Synthetic compound libraries are commercially available from a number of companies. A rare chemical library is available from Aldrich (Milwaukee, Wl). Combinatorial libraries of small organic molecules are available and can be prepared. Alternatively, libraries of natural compounds in the form of bacterial, fungal, plant and animal extracts are available or are readily producible by methods well known in the art. Additionally, natural and synthetically produced libraries and compounds are readily modified through conventional chemical, physical, and biochemical means.
As used herein, the term "small molecule" refers to a compound having molecular mass of less than 3000 Daltons, preferably less than 2000 or 1500, still more preferably less than 1000, and most preferably less than 600 Daltons. A "small organic molecule" is a small molecule that comprises carbon.
As used herein, the term "change in binding" or "change in activity" and the equivalent terms "difference in binding" or "difference in activity" refer to an at least 10% increase or decrease in binding, or signaling activity in a given assay.
As used herein, the term "conditions permitting the binding of Z9,E11-14:OAc to SlitOR5" refers to conditions of, for example, temperature, salt concentration, pH and protein concentration under which Z9,E1 1-14:OAc binds SlitOR5. Exact binding conditions will vary depending upon the nature of the assay, for example, whether the assay uses viable cells or only membrane fraction of cells. However, because SlitOR5 is a cell surface protein, and because Z9,E11-14:OAc normally interacts with SlitOR5 on the cell surface, favoured conditions will generally include physiological salt (90 mM) and pH (about 7.0 to 8.0). Temperatures for binding can vary from 4°C to 37°C, but will preferably be between room temperature and about 37°C. The concentration of Z9,E1 1-14:OAc and SlitOR5 polypeptide in a binding reaction will also vary, but will preferably be about 10 µM (e.g., in a reaction with radiolabeled tracer Z9,E11-14:OAc, where the concentration is generally below the Kd) to 1 mM (e.g., Z9,E11-14:OAc as competitor).
As used herein, the term "sample" refers to the source of molecules being tested for the presence of an agent that modulates binding to or signalling activity of a SlitOR5 polypeptide. A sample can be an environmental sample, a natural extract of animal, plant yeast or bacterial cells or tissues, a synthetic sample, or a conditioned medium from recombinant cells or a fermentation process. A "tissue" is an aggregate of cells that perform a particular function in an organism. The term "tissue" as used herein refers to cellular material from a particular physiological region. The cells in a particular tissue can comprise several different cell types. A non-limiting example of this would be brain tissue that further comprises neurons and glial cells, as well as capillary endothelial cells and blood cells, all contained in a given tissue section or sample. In addition to solid tissues, the term "tissue" is also intended to encompass non-solid tissues, such as blood.
As used herein, the term "membrane fraction" refers to a preparation of cellular lipid membranes comprising a SlitOR5 polypeptide. As the term is used herein, a "membrane fraction" is distinct from a cellular homogenate, in that at least a portion (i.e., at least 10%, and preferably more) of non-membrane-associated cellular constituents has been removed. The term "membrane associated" refers to those cellular constituents that are either integrated into a lipid membrane or are physically associated with a component that is integrated into a lipid membrane.
As used herein, the term "decrease in binding" refers to a decrease of at least 10% in the binding of Z9,E11-14:OAc or other agonist to a SlitOR5 polypeptide as measured in a binding assay as described herein.
As used herein, the term "second messenger" refers to a molecule, generated or caused to vary in concentration by the activation of a GPCR-like receptor that participates in the transduction of a signal from that receptor. Non-limiting examples of second messengers include cAMP, diacylglycerol, inositol triphosphates and intracellular calcium. The term "change in the level of a second messenger" refers to an increase or decrease of at least 10% in the detected level of a given second messenger relative to the amount detected in an assay performed in the absence of a candidate agent.
As used herein, the term "aequorin-based assay" refers to an assay for GPCR (or GPCR- like receptor) activity that measures intracellular calcium flux induced by activated receptors, wherein intracellular calcium flux is measured by the luminescence of aequorin expressed in the cell. Such a method is particularly valuable to quantify the Ca++ flux in cells expressing ion channel (like the OR - ORco heterodimers of insect olfaction).
As used herein, the term "binding" refers to the physical association of a ligand (e.g., Z9,E11-14:Oac or candidate agents as defined upper) with a receptor (e.g., SlitOR5). As the term is used herein, binding is "specific" if it occurs with an EC50 or a Kd of 100 nM or less, generally in the range of 100 µM to 1000 µM. Using odorant functional assay Ec50 found are usually around 10, 100 and even 1 mM, said signals may be considered specific (see Figure 3). Odorant binding may be considered specific if the EC50 or Kd is comprised between 1000µM and 1 nM or less.
As used herein, the term "EC50," refers to that concentration of an agent at which a given activity, including binding of Z9,E11-14:OAc or other ligand and a functional activity of a SlitOR5 polypeptide, is 50% of the maximum for that SlitOR5 activity measurable using the same assay. Stated differently, the "EC50" is the concentration of agent that gives 50% activation, when 100% activation is set at the amount of activity that does not increase with the addition of more agonist. It should be noted that the "EC50 of a candidate agent" will vary with the identity of the candidate agent; for example, the candidate agent can have EC50 values higher than, lower than or the same as Z9,E1 1-14:OAc. Therefore, where a candidate agent is used, one of the skill in the art can determine the EC50 for that agent according to conventional methods. The EC50 of a given candidate agent is measured by performing an assay for an activity of a fixed amount of SlitOR5 polypeptide in the presence of doses of the candidate agent that increase at least until the SlitOR5 response is saturated or maximal, and then plotting the measured SlitOR5 activity versus the concentration of the candidate agent used. As used herein, the term "Kd" is a dissociation constant or the ligand concentration at which half of the receptors are bound by the ligand at equilibrium.
As used herein, the term "IC50" is the concentration of an antagonist or inverse agonist that reduces the maximal activation of a SlitOR5 receptor by 50%.
As used herein, the term "detectably labelled" refers to the property of a molecule, e.g., Z9,E11-14:OAc or a candidate agent therefrom, that has a structural modification. Said modification is introduced through the incorporation or addition of a functional group. Said functional group (label) can be readily detected. Detectable labels include but are not limited to fluorescent compounds, isotopic compounds, chemiluminescent compounds, quantum dot labels.
Examples of radioisotopes which can be added to the structure of Z9,E11-14:OAc, or candidate agents, may be ¹⁴C, or ³H (as well as eventually also ¹²⁵l, ³⁵S). Examples of radioisotopes which can be incorporated to the structure of Z9,E11-14:OAc, or candidate agents, may be ³H or ¹⁴C.
The various means of detection include but are not limited to spectroscopic, photochemical, radiochemical, biochemical, immunochemical, or chemical means.
As used herein, the term "affinity tag" refers to a label, attached to a molecule of interest (e.g., a SlitOR5 polypeptide), that confers upon the labelled molecule the ability to be specifically bound by a reagent that binds the label. Affinity tags include, but are not limited to an epitope for an antibody (known as "epitope tags"), biotin, 6X His, Myc, FLAG and GST. Affinity tags can be used for the detection, as well as for the purification of the labelled species.
As used herein, the term "decrease in binding" refers to a decrease of at least 10% in the amount of binding detected in a given assay with a known or suspected modulator of SlitOR5 relative to binding detected in an assay lacking that known or suspected modulator.
As used herein, the term "delivering," when used in reference to an agent, means the addition of the agent to an assay mixture, or to a cell in culture. The term also refers to the administration of the agent to an animal. Such administration can be, for example, by injection (in a suitable carrier, e.g., sterile saline or water) or by puffing or other relevant route of drug administration.
As used herein, the term "transgenic animal" refers to any animal, usually an insect (*Drosophila* e.g.), in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or in vitro fertilization, but rather is directed to the introduction of a recombinant DNA molecule. This molecule may be integrated within a chromosome, or it may be extra-chromosomally replicating DNA. In the typical transgenic animals described herein, the transgene causes cells to express a recombinant form of one of the subject polypeptide, e.g. either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination and antisense techniques. As used herein, the term "antibody" is the conventional immunoglobulin molecule, as well as fragments thereof which are also specifically reactive with one of the subject polypeptides. It can alternatively encompass a heavy chain antibody (a.k.a. Camelid antibody). Antibodies can be fragmented using conventional techniques and the fragments screened for utility in the same manner as described herein below for whole antibodies. For example, F(ab)2 fragments can be generated by treating antibody with pepsin. The resulting F(ab)2 fragment can be treated to reduce disulfide bridges to produce Fab fragments. The antibody of the present invention is further intended to include bispecific, single-chain, and chimeric and humanized molecules having affinity for a polypeptide conferred by at least one CDR region of the antibody. In preferred embodiments, the antibodies, the antibody further comprises a label attached thereto and able to be detected, (e.g., the label can be a radioisotope, fluorescent compound, chemiluminescent compound, enzyme, or enzyme co- factor).
As used herein, the term "blocking agent" refers to any agent that stops or inhibits olfactory perception by the target. Said perception may be assayed using animal studies, ex vivo or in vitro studies simulating or representative for in vivo olfactory perception. With the expression "method of monitoring" is meant a method by which an infestation or a change in a population dynamic may be assessed, in said situation there exists already measurable indications or signs for the presence and/or development of said infestation or change in the population dynamic.
With the term ortholog is meant a gene with similar function to SlitOR5 in an evolutionary related species. With the term congeneric is meant a species of which phylogenetic affinities with one or some species in the genus *Spodoptera* Guenee can be demonstrated by a person skilled in the art.
As used herein "promoter" refers to the transcriptional control elements necessary for regulation of gene expression, including not only the basal promoter, but also any enhancers or transcription-factor binding sites necessary for receptor-regulated expression.

### BRIEF DESCRIPTION OF THE FIGURES

**Figure 1****.** Response spectrum from at1 olfactory sensory neurons of *w;UAS-SlitOr5; Or67d*^{GAL4} flies to 26 pheromone compounds (10 µg loaded in the stimulus cartridge). ***P*<0.01 vs. response to the solvent alone (Kruskall-Wallis ANOVA followed by a Dunn's post-hoc test). Error bars indicate standard error to the mean (*n*=7).
**Figure 2****.** Dose-response curve from at1 ORNs of *w;UAS-SlitOr5; Or67d*^{GAL4} flies to Z9,E11-14:OAc. ****P<*0.001, **P*<0.05 vs. response to the solvent alone. Error bars indicate SEM *(n=5).*
**Figure 3****.** Single-sensillum recordings from at1 ORNs of *w;UAS-SlitOr5; Or67d*^{GAL4} flies in response to: **A**. 10 µg of Z9,E11-14:OAc; **B**. 1 µg of Z9,E11-14:OAc; C. solvent (hexane). The black lines symbolise the duration of the stimulation (500 ms).

### DETAILED DESCRIPTION OF THE INVENTION

The invention relates to the insect transmembrane receptor, SlitOR5, and to the fact that Z9,E1 1-14:OAc is a natural ligand for said SlitOR5. The availability of SlitOR5 and its function in a critical insect behavior (attraction) provides the possibility to use this receptor for screening candidate agents able to interfere with SlitOR5 function and thus the corresponding behavior. Also, the interaction of SlitOR5 with Z9,E11-14:OAc is useful for screening assays for agents that modulate this interaction and thus the effect of Z9,E11-14:OAc on SlitOR5. The identification of candidate agents that bind to SlitOR5 or interfere with SlitOR5-Z9,E11-14:OAc binding provides opportunity for developing attracting lure based on single molecules or blends of at least 2 compounds, or for blocking SlitOR5 activity.

### SlitOR5 nucleotides/polypeptides

The polynucleotide and polypeptide sequence of *Spodoptera littoralis* SlitOR5 are presented herein in SEQ ID NO:1 and 2 .

The expression of recombinant polypeptides is well known in the art. Those skilled in the art can readily select vectors and expression control sequences for the expression of SlitOR5 polypeptides in eukaryotic or prokaryotic cells. SlitOR5 must be associated with cell membrane or detergents like synthetic liposomes in order to have binding or signalling function. Methods for the preparation of cellular membrane fractions are well known in the art, e.g., the method reported by Hubbard and Cohn, 1975, J. Cell Biol. 64: 461-479, In order to produce membranes comprising SlitOR5, one need only to apply such techniques to cells endogenously or recombinantly expressing SlitOR5. Alternatively, membrane-free SlitOR5 can be integrated into membrane preparations by dilution of detergent solution of the polypeptide.

According to the present invention, said SlitOR5 polypeptide may be a polypeptide having at least 60% identity or higher identity, such as 65%, 75%, 85%, 95% or even 100% to the polypeptide represented in SEQ ID NO:1. Preferably, said SlitOR5 polypeptide binds specifically a moth pheromone, preferably a *Spodoptera littoralis* pheromone. Preferably, said SlitOR5 polypeptide binds specifically Z9,E11-14:OAc.

Alternatively, said SlitOR5 polypeptide may be a fragment of the full length polypeptide as shown SEQ ID NO:1. Preferably, said fragment retains at least 50% of the binding activity and level of signaling activation when using Z9,E11-14:OAc.

According to the present invention, said SlitOR5 polypeptide may comprise one or more additions, insertions, deletions or substitutions relative to the sequence depictured in SEQ ID NO:1. Said SlitOR5 polypeptide may be a truncated SlitOR5 polypeptide; said SlitOR5 polypeptide may comprise additional sequences forming a SlitOR5 fusion protein, wherein said additional sequences may be chosen from the group consisting of glutathione-S-transferase (GST), maltose binding protein (MBP), alkaline phosphatase, thioredoxin, green fluorescent protein (GFP), histidine tags (e.g., 6X or greater His), or affinity tags (e.g., Myc tag, FLAG tag) sequences.

### Z9,E11-14:OAc

The structure of Z9,E1 1-14:OAc is well known by a skilled person. The skilled in the art may easily derive similar structures using multiple approaches which utilize diverse molecular descriptor values (i.e. chemical properties, e.g. molecular weight, vapor pressure, distance between atoms, functional group counts, three-dimensional relationships within molecules...) to describe structure and propose new molecules potentially active on the receptor. Such approaches may consist of, but are not restricted to Cerius2 (Accelrys Software Inc), Dragon (Talete), Maximum-Common-Substructure (MCS) and atom-pair (AP), or any optimized methods. An example of such design is illustrated in Boyles et al 2013 (Boyle et al. eLife 2013;2:e01120. DOI: 10.7554/eLife.01120). In addition, the skilled in the art may use SlitOR5 structure to design candidate agents which may be able to interact with SlitOR5 and may easily test if said equivalents are able to bind and/or modulate the SlitOR5 receptor. Z9,E11-14:OAc may be isolated from natural samples, or chemically synthesized.

Methods which can be used to quantify said Z9,E1 1-14:OAc may be, but are not limited to,
a) for extraction and purification: solvent extraction, oil extraction, vapour extraction, C0₂ supercritical extraction, liquid chromatography, distillation, gas chromatography;
b) for quantifying: gas chromatography, liquid chromatography, etc. coupled to mass spectrometry (e.g.).

A skilled person knows how to perform said methods.

Z9,E1 1-14:OAc may be used in purified form or used as composition. The amounts of Z9,E11-14:OAc necessary in a given binding or functional assay according to the invention will vary depending upon the assay, but will generally use 1 nM to 10 µM of labelled or 1 nM to 10mM of unlabelled Z9,E1 1-14:OAc per assay. If necessary for a given assay, Z9,E1 1-14:OAc can be labelled by incorporation or addition of radiolabeled labels as pointed above.

### Assays For The Identification Of Agonists/Antagonists/Inverse Agonists of the SlitOR5/ Z9,E11-14:OAc binding or signaling pathway

Agents that modulate the activity of SlitOR5 can be identified in a number of ways that take advantage of the interaction of said receptor with Z9,E1 1-14:OAc. For example, the ability to reconstitute SIitORS/Z9,E11-14:OAc binding either in vitro, on cultured cells or in vivo provides a target for identification of agents that disrupt that binding. Assays based on disruption of binding can identify agents, such as small organic molecules, from libraries or collections of such molecules. Alternatively, such assays can identify agents in samples or extracts from natural sources, including plant, fungal or bacterial extracts or even human tissue samples. Modulators of SlitOR5/Z9,E11-14:OAc binding can then be screened using a binding assay or a functional assay that measures downstream signaling/cell electric response through the said receptor. Both binding assays and functional assays are validated using Z9,E11-14:OAc.

Another approach that uses the SlitOR5/Z9,E11-14:OAc interaction more directly to identify agents that modulate SlitOR5 function measures changes in SlitOR5 downstream signaling induced by candidate agents. These functional assays can be performed in isolated cell membrane fractions or on cells expressing the receptor on their surfaces or in organisms expressing SlitOR5.

Functional assays, in which a signaling activity of SlitOR5 is measured, may be used:
a) For agonist screening, cells expressing SlitOR5 or membranes prepared from them or organisms expressing SlitOR5 are incubated/stimulated with a candidate agent, and a signaling activity of SlitOR5 is measured. The assays are validated using Z9,E1 1-14:OAc as agonist, and the activity induced by compounds that modulate receptor activity is compared to that induced by Z9,E11-14:OAc. An agonist or partial agonist will have a maximal biological activity corresponding to at least 10% of the maximal activity of Z9,E1 1-14:OAc when the agonist or partial agonist is present at 100 µM or less, and preferably will have 50%, 75%, 100% or more, including 2-fold, 5-fold, 10-fold or more activity than Z9,E1 1-14:OAc.
b) For antagonist or inverse agonist screening, cells expressing SlitOR5 or membranes isolated from them or organisms expressing SlitOR5 are assayed for signaling activity in the presence of Z9,E11- 14:OAc with and without a candidate agent. Antagonists or inverse agonists will reduce the level of Z9,E11-14:OAc-stimulated receptor activity by at least 10%, relative to reactions lacking the antagonist or inverse agonist.
c) For inverse agonist screening, cells expressing constitutive SlitOR5 activity or membranes isolated from them or organisms expressing SlitOR5 are used in a functional assay that measures an activity of the receptor or the animal behavior in the presence and in the absence of a candidate agent. Inverse agonists are those compounds that reduce the constitutive activity of the receptor or the animal behavior by at least 10%. Overexpression of SlitOR5 may lead to constitutive activation. SlitOR5 can be overexpressed by placing it under the control of a strong constitutive promoter, e.g., the CMV early promoter.

Functional assays of receptor activity may be used, that may consist of expression of SlitOR5 in yeast, bacteria, cultured cells (such as HEK, HeLa, CHO, COS, Sf9, S2, HiFive), *Xenopus* oocytes or in an organism (such as *Drosophila melanogaster*).

### i. Downstream Pathway Activation Assays:

### a. Calcium flux - The Aequorin-based Assay

The aequorin assay takes advantage of the responsiveness of mitochondrial or cytoplasmic apoaequorin to intracellular calcium release or calcium flux (entrance) induced by the activation of membrane receptors. Briefly, SlitOR5 and SlitORco-expressing clones are transfected to coexpress mitochondrial or cytoplasmic apoaequorin and Gcc16 or G-olf. Cells are incubated with 5 µM Coelenterazine H or derivates (Molecular Probes) for 4 hours at room temperature, washed in DMEM-F12 culture medium and resuspended at a concentration of 0.5 x 10⁶ cells/ml.

Cells are then mixed with test candidate agent and light emission by the aequorin is recorded with a luminometer for 30 sec. Results are expressed as Relative Light Units (RLU). Controls include assays using membranes isolated from cells not expressing SlitOR5 (mock-transfected), in order to exclude possible non-specific effects of the candidate agent.

Aequorin activity or intracellular calcium levels are "changed" if light intensity increases or decreases by 10% or more in a sample of cells, expressing a SlitOR5 polypeptide and treated with a candidate agent, relative to a sample of cells expressing the SlitOR5 polypeptide but not treated with the candidate agent or relative to a sample of cells not expressing the SlitOR5 polypeptide (mock-transfected cells) but treated with the candidate agent. When performed in the absence of Z9,E11-14:OAc, the assay can be used to identify an agonist or inverse agonist of SlitOR5 activity. When the assay is performed in the presence of Z9,E11-14:OAc, it can be used to assay for an antagonist.

### b) Calcium imaging - Fluo3, 4, Fura2, and Calcium3 (Molecular Device) based-assay

Fluorescence-based assays take advantage of calcium fluxes triggered by receptor activation: either calcium entrance through ORco for instance or calcium release from endoplasmic reticulum. Some fluorophores including but not limited to Fluo3, Fluo4 and Fura2 (Molecular Probes) and Calcium3 kit series (Molecular Device) are known to bind calcium. Such fluorophore-calcium complexes emit fluorescence at respective specific wavelength. Thereby, upon activation of a membrane receptor, calcium released from endoplasmic reticulum or entered through ORco binds to fluorophore leading to specific fluorescence emission. Typically, SlitOR5 is overexpressed in cells with or without SlitORco (depending on the expression system) and cells are incubated for 30 to 60 minutes with a solution of 1 to 8 µM fluorophore at 37°C. After thorough washing with saline buffer, 50 µI of the same buffer is poured in each well-containing cells (6 to 1536). Tested agonists are then injected onto such loaded-cells and activation of SlitOR5 is followed by fluorescence measurement. Intracellular calcium levels are modified if fluorescence intensity increases or decreases by 10% or more in a sample of cells, expressing a SlitOR5 polypeptide and treated with a candidate agent, relative to a sample of cells expressing the SlitOR5 polypeptide but not treated with the candidate agent or relative to a sample of cells not expressing the SlitOR5 polypeptide (mock-transfected cells) but treated with the candidate agent.

### c) Depolarization/hyperpolarization membrane assay (DiBac fluorophore for instance).

The principle of this assay is to follow depolarization of cell membrane. For instance, the anionic probe DiBAC4(3) partitions between intra- and extracellular compartments in a membrane potential-dependent manner. With increasing membrane potential (depolarization), the probe further partition into the cell resulting in an increase of fluorescence. Conversely, hyperpolarization leads to a decrease of fluorescence due to a dye extrusion.

The DiBAC4(3) probe is excited with a wavelength of 488 nm, and emits at a wavelength of 540 nm.

On the day of the experiment, add the glucose to the assay buffer (saline buffer) to a final concentration of 10 mM and the DiBAC4(3) probe to a final concentration of 5 µM.

Maintain the assay buffer at 37°C. Remove the cell culture medium and rinse twice each well containing SlitOR5/SlitORco-overexpressing cells with 200 µI of pre-heated assay buffer. Place 180 µI of Assay buffer containing DiBAC4(3) and incubate cells for 30 min at the appropriate temperature. Cell plates will be ready for assay after these 30 min incubation. Collect baseline for 2 min prior any addition. Add 20 µI of candidate agents to the appropriate well and collect the data for an additional 25 min. Membrane polarization is "changed" if fluorescence intensity increases or decreases by 10% or more in a sample of cells, expressing a SlitOR5 polypeptide and treated with a candidate agent, relative to a sample of cells expressing the SlitOR5 polypeptide but not treated with the candidate agent or relative to a sample of cells not expressing the SlitOR5 polypeptide (mock-transfected cells) but treated with the candidate agent.

### d. Adenylate Cyclase Assay:

Assays for adenylate cyclase activity are described by Kenimer and Nirenberg, 1981 , Mol. Pharmacol. 20: 585-591 , incorporated herein by reference. That assay is a modification of the assay taught by Solomon et al., 1974, Anal. Biochem. 58: 541 -548, also incorporated herein by reference. Briefly, 100 µI reactions contain 50 mM Tris-Hcl (pH 7.5), 5 mM MgCI2, 20 mM creatine phosphate (disodium salt), 10 units (71 µg of protein) of creatine phosphokinase, 1 mM cc-³²P-ATP (tetrasodium salt, 2 µθí), 0.5 mM cyclic AMP, G-³H-labelled cyclic AMP (approximately 10,000 cpm), 0.5 mM Ro20-1724, 0.25% ethanol, and 50-200 µg of protein homogenate to be tested (i.e., homogenate from cells expressing or not expressing a SlitOR5 polypeptide, treated or not treated with Z9,E11-14:OAc with or without a candidate agent). Reaction mixtures are generally incubated at 37°C for 6 minutes. Following incubation, reaction mixtures are deproteinized by the addition of 0.9 ml of cold 6% trichloroacetic acid. Tubes are centrifuged at 1800 x g for 20 minutes and each supernatant solution is added to a Dowex AG50W-X4 column. The cAMP fraction from the column is eluted with 4 ml of 0.1 mM imidazole-HCI (pH 7.5) into a counting vial. Assays should be performed in triplicate. Control reactions should also be performed using protein homogenate from cells that do not express a SlitOR5 polypeptide.

Assays should be performed using cells or extracts of cells expressing SlitOR5/SlitORco, treated or not treated with a candidate agent, compared with said cells or extracts of cells expressing SlitOR5/SlitORco, treated or not treated with Z9,E11-14:OAc. Control reactions should be performed using mock-transfected cells, or extracts from them in order to exclude possible non-specific effects of some candidate agents.

Adenylate cyclase activity is "changed" if it increases or decreases by 10% or more in a sample taken from cells treated with a candidate agent of SlitOR5 activity, relative to a similar sample of cells not treated with the candidate agent or relative to a sample of cells not expressing the SlitOR5 polypeptide (mock-transfected cells) but treated with the candidate agent. Alternatively, a decrease of activity by 10% or more by the candidate agent of SlitOR5 in a sample treated with a reference compound may be tested.

### e. cAMP/cGMP Assays:

Intracellular cAMP/cGMP is measured using a cAMP/cGMP radioimmunoassay (RIA) or cAMP/cGMP binding protein according to methods widely known in the art. For example, Horton and Baxendale, 1995, Methods Mol. Biol. 41 : 91 -105, describes an RIA for cAMP.

A number of kits for the measurement of cAMP/cGMP are commercially available, such as the High Efficiency Fluorescence Polarization-based homogeneous assay marketed by LJL Biosystems and NEN Life Science Products. Control reactions should be performed using extracts of mock-transfected cells to exclude possible non-specific effects of some candidate agents.

Assays should be performed using cells or extracts of cells expressing SlitOR5/SlitORco, treated or not treated with a candidate agent, compared with said cells or extracts of cells expressing SlitOR5/SlitORco, treated or not treated with Z9,E11-14:OAc. Control reactions should be performed using mock-transfected cells, or extracts from them in order to exclude possible non-specific effects of some candidate agents.

The level of cAMP is "changed" if the level of cAMP/cGMP detected in cells, expressing a SlitOR5 polypeptide and treated with a candidate agent of SlitOR5 activity (or in extracts of such cells), using the RIA-based assay of Horton and Baxendale, 1995, supra, increases or decreases by at least 10% relative to the cAMP level in similar cells not treated with the candidate agent.

### f. Phospholipid breakdown, DAG production and Inositol Triphosphate levels:

Receptors that activate the breakdown of phospholipids can be monitored for changes due to the activity of known or suspected modulators of SlitOR5 by monitoring phospholipid breakdown, and the resulting production of second messengers DAG and/or inositol triphosphate (IP3). Methods of measuring each of these are described in Phospholipid Signalling Protocols, edited by Ian M. Bird. Totowa, NJ, Humana Press, 1998, which is incorporated herein by reference. See also Rudolph et al., 1999, J. Biol. Chem. 274: 1 1824- 1 1831, which also describes an assay for phosphatidylinositol breakdown. Assays should be performed using cells or extracts of cells expressing SlitOR5/SlitORco, treated or not treated with a candidate agent, compared with said cells or extracts of cells expressing SlitOR5/SlitORco, treated or not treated with Z9,E11-14:OAc. Control reactions should be performed using mock-transfected cells, or extracts from them in order to exclude possible non-specific effects of some candidate agents. Phosphatidylinositol breakdown, and diacylglycerol and/or inositol triphosphate levels are "changed" if they increase or decrease by at least 10% in a sample from cells expressing a SlitOR5 polypeptide and treated with a candidate agent relative to the level observed in a sample from cells expressing a SlitOR5 polypeptide that is not treated with the candidate agent and relative to the level observed in a sample from cells expressing a SlitOR5 polypeptide that is treated with Z9,E11-14:Oac.

### ii. Transcriptional reporters for downstream pathway activation:

The intracellular signal initiated by binding of an agonist to a receptor, e.g., SlitOR5, sets in motion a cascade of intracellular events, the ultimate consequence of which is a rapid and detectable change in the transcription and/or translation of one or more genes. The activity of the receptor can therefore be monitored by measuring the expression of a reporter gene driven by control sequences responsive to SlitOR5 activation.

By selecting promoters that are responsive to the intracellular signals resulting from agonist binding, and operatively linking the selected promoters to reporter genes whose transcription, translation or ultimate activity is readily detectable and measurable, the transcription based reporter assay provides a rapid indication of whether a given receptor is activated. Reporter genes such as luciferase, Chloramphenicol Acetyl Transferase (CAT), Green Fluorescent Protein (GFP), β-lactamase or β-galactosidase are well known in the art, as are assays for the detection of their products.

Genes particularly well suited for monitoring receptor activity are the "immediate early" genes, which are rapidly induced, generally within minutes of contact between the receptor and the effector protein or ligand. The induction of immediate early gene transcription does not require the synthesis of new regulatory proteins. In addition to rapid responsiveness to ligand binding, characteristics of preferred genes useful to make reporter constructs include: low or undetectable expression in quiescent cells; induction that is transient and independent of new protein synthesis; subsequent shut-off of transcription requires new protein synthesis; and mRNAs transcribed from these genes have a short half-life. It is preferred, but not necessary that a transcriptional control element have all of these properties for it to be useful. An example of a gene that is responsive to a number of different stimuli is the c-fos proto-oncogene. The c-fos gene is activated in a protein-synthesis-independent manner by growth factors, hormones, differentiation-specific agents, stress, and other known inducers of cell surface proteins. The induction of c-fos expression is extremely rapid, often occurring within minutes of receptor stimulation. This characteristic makes the c-fos regulatory regions particularly attractive for use as a reporter of receptor activation.

The c-fos regulatory elements include: a TATA box that is required for transcription initiation; two upstream elements for basal transcription, and an enhancer, which includes an element with dyad symmetry and which is required for induction by phorbol ester 12-0-tetradecanoylphorbol- -acetate (TPA), serum, Epidermal Growth Factor (EGF), and PMA.

The 20 bp c-fos transcriptional enhancer element located between -317 and -298 bp upstream from the c-fos mRNA cap site, is essential for serum induction in serum starved NIH 3T3 cells. One of the two upstream elements is located at -63 to -57 and it resembles the consensus sequence for cAMP regulation.

The transcription factor CREB (cyclic AMP responsive element binding protein) is, as the name implies, responsive to levels of intracellular cAMP. Therefore, the activation of a receptor that signals via modulation of cAMP levels can be monitored by measuring either the binding of the transcription factor, or the expression of a reporter gene linked to a CREB- binding element (termed the CRE, or cAMP response element). The DNA sequence of the CRE is TGACGTCA. Reporter constructs responsive to CREB binding activity are described in U.S. Patent No. 5,919,649.

Other promoters and transcriptional control elements, in addition to the c-fos elements and CREB-responsive constructs, include the vasoactive intestinal peptide (VIP) gene promoter (cAMP responsive; Fink et al., 1988, Proc. Natl. Acad. Sci. 85:6662-6666); the somatostatin gene promoter (cAMP responsive; Montminy et al., 1986, Proc. Natl. Acad. Sci. 8.3:6682-6686); the proenkephalin promoter (responsive to cAMP, nicotinic agonists, and phorbol esters; Comb et al., 1986, Nature 323:353-356); the phosphoenolpyruvate carboxylase (PEPCK) gene promoter (cAMP responsive; Short et al., 1986, J. Biol. Chem. 261 :9721 -9726). Additional examples of transcriptional control elements that are responsive to changes in GPCR or GPCR-like activity include, but are not limited to those responsive to the AP-1 transcription factor and those responsive to NF-κB activity. The consensus AP-1 binding site is the palindrome TGA(C/G)TCA (Lee et al., 1987, Nature 325: 368-372; Lee et al., 1987, Cell 49: 741 -752). The AP-1 site is also responsible for mediating induction by tumor promoters such as the phorbol ester 12-0-tetradecanoylphorbol-acetate (TPA), and are therefore sometimes also referred to as a TRE, for TPA-response element. AP-1 activates numerous genes that are involved in the early response of cells to growth stimuli. Examples of AP-1 - responsive genes include, but are not limited to the genes for Fos and Jun (which proteins themselves make up AP-1 activity), Fos-related antigens (Fra) 1 and 2, IκBα, ornithine decarboxylase, and annexins I and II. The NF-κB binding element has the consensus sequence GGGGACTTTCC. A large number of genes have been identified as NF-κB responsive, and their control elements can be linked to a reporter gene to monitor GPCR activity. A small sample of the genes responsive to NF-κB includes those encoding IL-1 β, TNF-cc, CCR5, P-selectin, Fas ligand, GM-CSF and IκBα. Vectors encoding NF-KB-responsive reporters are also known in the art or can be readily made by one of skill in the art using, for example, synthetic NF-κB elements and a minimal promoter, or using the NF-KB-responsive sequences of a gene known to be subject to NF-κB regulation. Further, NF-κB responsive reporter constructs are commercially available from, for example, CLONTECH.

A given promoter construct should be tested by exposing SlitOR5/SlitORco-expressing cells, transfected with the construct, to Z9,E11-14:OAc. An increase of at least two-fold in the expression of reporter in response to Z9,E1 1-14:OAc indicates that the reporter is an indicator of SlitOR5 activity.

In order to assay SlitOR5 activity with Z9,E11-14:OAc-responsive transcriptional reporter construct, cells that stably express SlitOR5/SlitORco polypeptides are stably transfected with the reporter construct. To screen for agonists, untreated cells are exposed to candidate agents, or exposed to Z9,E11-14:OAc, and expression of the reporter is measured. The Z9,E11-14:OAc- treated cultures serve as a standard for the level of transcription induced by a known agonist. An increase of at least 10% in reporter expression in the presence of a candidate agent compare to reporter expression in the absence of any modulator indicates that the candidate is a modulator of SlitOR5 activity. An agonist will induce at least as much, and preferably the same amount or more reporter expression than the Z9,E11-14:OAc. Partial agonists may activate the receptor less compared to Z9,E1 1-14:OAc. This approach can also be used to screen for inverse agonists where cells express a SlitOR5 polypeptide at levels such that there is an elevated basal activity of the reporter in the absence of Z9,E11-14:OAc or other agonists. A decrease in reporter activity of 10% or more in the presence of a candidate agent, relative to its absence, indicates that the compound is an inverse agonist. To screen for antagonists, the cells expressing SlitOR5 and carrying the reporter construct are exposed to Z9,E11-14:OAc (or another agonist) in the presence and absence of a candidate agent. A decrease of 10% or more in reporter expression in the presence of said candidate agent, relative to the absence of said candidate agent, indicates that the candidate is an antagonist of SlitOR5 activity. Controls for transcription assays include cells not expressing SlitOR5 but carrying the reporter construct, as well as cells with a promoterless reporter construct. Compounds that are identified as modulators of SlitOR5-regulated transcription should also be analyzed to determine whether they affect transcription driven by other regulatory sequences and by other receptors, in order to determine the specificity and spectrum of their activity. The transcriptional reporter assay, and most cell-based assays, are well suited for screening chemical libraries of chemical compounds for those that modulate SlitOR5 activity. The libraries can be, for example, libraries from natural sources, e.g., plants, animals, bacteria, etc.

### iii. Receptor internalization

Any of the assays of receptor activity, including calcium flux, membrane polarization, melanophore assay, the GTP-binding, GTPase, adenylate cyclase, cAMP, phospholipid-breakdown, diacylglyceorl, inositol triphosphate, PKC, PKA, kinase, receptor internalization and transcriptional reporter assays, can be used to determine the presence of an agent in a sample, e.g., a tissue sample, that affects the activity of the SlitOR5 receptor molecule. To do so, SlitOR5 polypeptide is assayed for activity in the presence and in the absence of the sample or an extract of the sample. An increase in SlitOR5 activity in the presence of the sample or extract relative to the absence of the sample indicates that the sample contains an agonist of the receptor activity. A decrease in receptor activity in the presence of Z9,E11-14:OAc or another agonist and the sample, relative to receptor activity in the presence of Z9,E11-14:OAc alone, indicates that the sample contains an antagonist of SlitOR5 activity. If desired, samples can then be fractionated and further tested to isolate or purify the agonist or antagonist. The amount of increase or decrease in measured activity necessary for a sample to be said to contain a modulator depends upon the type of assay used. Generally, a 10% or greater change (increase or decrease) relative to an assay performed in the absence of a sample indicates the presence of a modulator in the sample. One exception is the transcriptional reporter assay, in which at least a two-fold increase or 10% decrease in signal is necessary for a sample to be said to contain a modulator. It is preferred that an agonist stimulates at least 50%, and preferably 75% or 100% or more, e.g., 2-fold, 5-fold, 10-fold or greater receptor activation than Z9,E1 1-14:OAc.

### iv. Electrophysiological recording

### a. Two-voltage clamp assay on Xenopus oocytes

Typically (Liu et al., Insect Biochem Mol Biol 2013, 43(8):747-754), mature oocytes (stage V-VII) are treated with 2 mg/mL collagenase S-1 in washing buffer (96 mm NaCl, 2 mm KC1, 5 mm MgC12 and 5 mm N-2-hydroxyethylpiperazine-N#-2-ethanesulfonic acid [HEPES] [pH 7.6]) for 1-2 h at room temperature. Oocytes are then microinjected with complementary RNAs or plasmids to co-express SlitOR5/SlitORco. After injection, oocytes are incubated for 3-5 days at 18°C in Ringer's solution (96 mm NaCl, 2 mm KCl, 5 mm MgC12, 0.8 mm CaC12, and 5 mm HEPES [pH 7.6]) supplemented with 5% dialyzed horse serum, 50 mg/mL tetracycline, 100 mg/mL streptomycin, and 550 mg/mL sodium pyruvate. Whole-cell currents are recorded from the injected *Xenopus* oocytes by using a 2-electrode voltage clamp at a holding potential of-80 mV upon stimulation with candidate agents (10⁻⁹ to 10⁻³M).

### b. Electroantennography and single sensillum recording

SlitOR5 can be heterologously expressed in vivo in an organism, for example *Drosophila melanogaster,* using transgenesis (e.g. the Gal4-UAS system *in D. melanogaster*). SlitOR5 can be expressed in a large part of the endogenous olfactory sensory neurons (OSNs) of the organism (for instance using the ORco promotor) or in a given subset of the OSN population. The modified responses of the antenna or of the targeted OSNs are then recorded using, respectively, electroantennography (EAG) and single sensillum recording (SSR). Some *D*. *melanogaster* mutant lines may be used to replace a native *Drosophila* OR with SlitOR5, such as the ab3A mutant line which allows for expression of SlitOR5 in ORNs usually expressing DmelOR22a (Hallem et al., Cell 2004, 117(7):965-979), and the DmelOr67dGAL4 mutant knock-in line, which allows for expression of SlitOR5 in place of DmelOR67d, in the unique OSN of the at1 *Drosophila* sensilla (Kurtovic et al. Nature 2007, 446(7135):542-546). The candidate agent activity is measured as the antenna depolarization (EAG) or the number of action potential (SSR) generated by stimulation of an air flow containing the candidate agent (typically from 0,1 µg to 100 µg of the candidate agent loaded in the stimulation cartridge). Depolarization and action potential number are compared, respectively, to depolarization and action potential number generated by the agent solvent alone.

### v. Animal behavior

When SlitOR5 is heterologously expressed in vivo in an organism, for example *Drosophila melanogaster*, activity of SlitOR5 can also be measured using behavioral assays. Such assays may be, but are not restricted to, attraction assay or choice assay, and may use, but are not restricted to the use of, wind tunnel, olfactometer, Y-tube, locomotion compensator, Petri dish, behavior arena... The set-up can be dynamic with an air flow containing or not the candidate agent or be static without air flow, the candidate agent being place on one side of the set-up. Different parameters of the animal behavior are registered, such as, but not restricted to, time to generate active behavior, orientation to the source, distance, preference index... The activity of organism expressing SlitOR5 is compared to wild type animals and to the activity induced by the agent solvent.
Other functional assays include, for example, microphysiometer or biosensor assays.

### Assays to Identify Agonists/Antagonists/Inverse Agonists among Ligands of SlitOR5

The discovery that Z9,E1 1-14:OAc is a ligand of the SlitOR5 receptor permits screening assays to identify agonists, antagonists and inverse agonists of receptor activity. The screening assays will have two general approaches.

### 1) Ligand binding assays, in which cells expressing SlitOR5, membrane extracts from such cells, or immobilized lipid membranes comprising SlitOR5 or organisms expressing SlitOR5 are exposed to a candidate agent.

Binding of a compound may be classified in 3 main categories: competitive binding, non-competitive binding and uncompetitive binding. A competitive binding compound resembles a second (reference) compound and binds to the same binding pocket of a target molecule (here receptor). Upon addition, the competitive binding compound displaces said second compound from said target. A non-competitive binding compound does not bind to the same binding pocket of the target molecule as a second (reference) compound but may interact with the effect of said second compound on said target molecule. The second compound is not displaced upon addition of the non-competitive binding compound. An uncompetitive-binding compound binds to the target molecule when a second compound is already bound. Cooperative binding means that a compound facilitates the binding of another compound which may be a reference compound. The cooperative effect is thus seen in the analysis of the Kd of said other compound.

### 2) Ligand binding and displacement assays.

One can use SlitOR5 polypeptides expressed on a cell, or isolated membranes containing receptor polypeptides, along with Z9,E11-14:OAc in order to screen for compounds that inhibit the binding of Z9,E1 1-14:OAc to SlitOR5. When identified in an assay that measures binding or Z9,E1 1-14:OAc displacement alone, candidate agents will have to be subjected to functional testing to determine whether they act as agonists, antagonists or inverse agonists. For displacement experiments, cells expressing a SlitOR5 polypeptide (generally 25,000 cells per assay or 1 to 100 µg of membrane extracts) are incubated in binding buffer (e.g., 50 mM Hepes pH 7.4; 1 mM CaC1₂; 0.5% Bovine Serum Albumin (BSA) Fatty Acid-Free; and 0,5 mM MgCh for 1.5 hrs (at, for example, 27°C) with labelled Z9,E1 1-14:OAc in the presence or in the absence of increasing concentrations of a candidate agent. To validate and calibrate the assay, control competition reactions using increasing concentrations of unlabelled Z9,E11-14:OAc can be performed. After incubation, cells are washed extensively, and bound, labelled Z9,E11-14:OAc is measured as appropriate for the given label (e.g., scintillation counting, enzyme assay, fluorescence, etc.). A decrease of at least 10% in the amount of labelled Z9,E1 1-14:OAc bound in the presence of candidate agent indicates displacement of binding by the candidate agent. Candidate agents are considered to bind specifically in this or other assays described herein if they displace 50% of labelled Z9,E1 1-14:OAc (sub-saturating Z9,E1 1-14:OAc dose) at a concentration of 100 µM or less (i.e., EC50 is 100 µM or less). Alternatively, binding or displacement of binding can be monitored by surface plasmon resonance (SPR). Surface plasmon resonance assays can be used as a quantitative method to measure binding between two molecules by the change in mass near an immobilized sensor caused by the binding or loss of binding of Z9,E1 1-14:OAc from the aqueous phase to a SlitOR5 polypeptide immobilized in a membrane on the sensor. This change in mass is measured as resonance units versus time after injection or removal of Z9,E11-14:OAc or candidate agent and is measured using a Biacore Biosensor (Biacore AB). SlitOR5 can be immobilized on a sensor chip (for example, research grade CM5 chip; Biacore AB) in a thin film lipid membrane.

SPR can assay for modulators of binding in at least two ways. First, Z9,E1 1-14:OAc can be pre-bound to immobilized SlitOR5 polypeptide, followed by injection of candidate agent at approximately 10 µI/min flow rate and a concentration ranging from 1 nM to 1000 µM, preferably about 100 µM. Displacement of the bound Z9,E11-14:OAc can be quantitated, permitting detection of modulator binding. Alternatively, the membrane-bound SlitOR5 polypeptide can be pre-incubated with candidate agent and challenged with Z9,E11- 14:OAc. A difference in Z9,E1 1-14:OAc binding to the SlitOR5 exposed to modulator relative to that on a chip not pre-exposed to modulator will demonstrate binding. In either assay, a decrease of 10% or more in the amount of Z9,E1 1-14:OAc bound is in the presence of candidate agent, relative to the amount of Z9,E1 1-14:OAc bound in the absence of candidate agent indicates that the candidate agent inhibits the interaction of SlitOR5 and Z9,E1 1-14:OAc. Biacore system can be plugged to a system identifying candidate agent such as mass spectrometry, or gas chromatography.

Another method of measuring inhibition of binding of Z9,E11-14:OAc to SlitOR5 uses fluorescence resonance energy transfer (FRET). FRET is a quantum mechanical phenomenon that occurs between a fluorescence donor (D) and a fluorescence acceptor (A) in close proximity to each other (usually < 100 A of separation) if the emission spectrum of D overlaps with the excitation spectrum of A. The molecules to be tested, e.g., Z9,E11-14:OAc and a SlitOR5 polypeptide, are labelled with a complementary pair of donor and acceptor fluorophores. While close to each other due to SlitOR5:Z9,E11-14:OAc interaction, fluorescence emitted upon excitation of the donor fluorophore will have a different wavelength than that emitted in response to that excitation wavelength when the molecules are not bound, providing for quantitation of bound versus unbound polypeptides by measurement of emission intensity at each wavelength. Donor:Acceptor pairs of fluorophores with which to label the target molecules are well known in the art.

A variation on FRET uses fluorescence quenching to monitor molecular interactions. One molecule in the interacting pair can be labelled with a fluorophore, and the other with a molecule that quenches the fluorescence of the fluorophore when brought into close apposition with it. A change in fluorescence upon excitation is indicative of a change in the association of the molecules tagged with the fluorophore: quencher pair. Generally, an increase in fluorescence of the labelled SlitOR5 polypeptide is indicative that Z9,E11-14:OAc bearing the quencher has been displaced. For quenching assays, a 10% or greater increase in the intensity of fluorescent emission in samples containing a candidate agent, relative to samples without the candidate agent, indicates that the candidate agent inhibits SlitOR5: Z9,E1 1-14:OAc interaction.

Bioluminescence Resonance Energy Transfer (BRET) is a system for monitoring intermolecular interactions in vivo. The assay is based on non-radiative energy transfer between fusion proteins containing Renilla luciferase (Rluc) and e.g. Yellow Fluorescent Protein (YPF) or Green Fluorescent Protein (GFP). The BRET signal is generated by the oxidation of a coelenterazine derivative substrate. Said system may apply a cell-permeable and non-toxic coelenterazine derivative substrate DeepBleuC™ (DBC) and a mutant of the Green Fluorescent Protein (GFP) as acceptor. When the donor and acceptor are in close proximity the energy resulting from the catalytic degradation of the DBC is transferred from Rluc to GFP which will then emit fluorescence as its characteristic wavelength. This method allows higher distance between the two tested molecules and is fluorophore-angle independent.

In addition to the surface plasmon resonance and FRET and BRET methods, fluorescence polarization measurement is useful to quantitate Z9,E11-14:OAc-receptor binding. The fluorescence polarization value for a fluorescently-tagged molecule depends on the rotational correlation time or tumbling rate. Protein complexes, such as those formed by SlitOR5 associating with a fluorescently labelled Z9,E11-14:OAc, have higher polarization values than uncomplexed, labelled Z9,E1 1-14:OAc. The inclusion of a candidate inhibitor of the SlitOR5: Z9,E11-14:OAc interaction results in a decrease in fluorescence polarization, relative to a mixture without the candidate inhibitor, if the candidate inhibitor disrupts or inhibits the interaction of SlitOR5 with Z9,E1 1-14:OAc. Fluorescence polarization is well suited for the identification of small molecules that disrupt the formation of polypeptide or protein complexes. A decrease of 10% or more in fluorescence polarization in samples containing a candidate agent, relative to fluorescence polarization in a sample lacking the candidate agent, indicates that the candidate agent inhibits SlitOR5: Z9,E11-14:OAc interaction.

Another alternative for monitoring SlitOR5: Z9,E11-14:OAc interactions uses a biosensor assay. ICS biosensors have been described by AMBRI (Australian Membrane Biotechnology Research Institute; http//www.ambri. com.au/). In this technology, the association of molecules such as SlitOR5 and Z9,E11-14:OAc, is coupled to the closing of gramacidin-facilitated ion channels in suspended membrane bilayers and thus to a measurable change in the admittance (similar to impedence) of the biosensor. This approach is linear over six orders of magnitude of admittance change and is ideally suited for large scale, high throughput screening of small molecule combinatorial libraries. A 10% or greater change (increase or decrease) in admittance in a sample containing a candidate agent, relative to the admittance of a sample lacking the candidate agent, indicates that the candidate agent inhibits the interaction of SlitOR5 and Z9,E11-14:OAc.

It is important to note that in assays of ligand-protein interaction, it is possible that a modulator of the interaction need not necessarily to interact directly with the domain(s) of the proteins that physically interact. It is also possible that a modulator will interact at a location removed from the site of ligand-protein interaction and cause, for example, a conformational change in the SlitOR5 polypeptide. Modulators (inhibitors or agonists) that act in this manner are nonetheless of interest as agents to modulate the activity of SlitOR5. Any of the binding assays described can be used to determine the presence of an agent in a sample, e.g., a tissue sample, that binds to the SlitOR5 receptor molecule, or that affects the binding of Z9,E11-14:OAc to the receptor. To do so, SlitOR5 polypeptide is reacted with Z9,E1 1-14:OAc in the presence or in the absence of the sample, and Z9,E11-14:OAc binding is measured as appropriate for the binding assay being used. A decrease of 10% or more in the binding of Z9,E11-14:OAc indicates that the sample contains an agent that modulates Z9,E1 1-14:OAc binding to the receptor polypeptide.

The methods of the present invention may be applied on protein chips. Said protein chip may be, but is not limited to, a glass slide or a nitrocellulose membrane. Array-based methods for protein chips are well known in the art.

### Population monitoring and mass trapping Based upon the Interaction of SlitOR5 and Ligands/Agonists/Antagonists/Inverse Agonists

The interaction of SlitOR5 with its ligands/Agonists/Antagonists/Inverse Agonists can be used as the basis of monitoring and controlling infestation of *S. littoralis* based on SlitOR5-related behaviours (attraction and trapping). This can have several different forms. Standardized trapping and mass trapping, "attract and kill" or mark "and recapture" assays as well as mating disruption (via disruption of pheromone detection at the level of the receptor) can be designed using ligands/Agonists/Antagonists/Inverse Agonists to 1) estimate the population size, its growth and the necessity of using population control systems, and 2) control populations.

### Qualitative assays

Assays that evaluate whether or not the SlitOR5 polypeptide or the mRNA encoding it are wild-type or not can be used diagnostically. In order to diagnose mutation in the olfactory function, RNA isolated from a sample is used as a template for PCR amplification of SlitOR5. The amplified sequences are then either directly sequenced using standard methods, or are first cloned into a vector, followed by sequencing. A difference in the sequence that changes one or more encoded amino acids relative to the sequence of wild-type SlitOR5 can be diagnostic of a mutation characterized by change of SlitOR5 signaling. It can be useful, when a change in coding sequence is identified in a sample, to express the variant receptor or ligand and compare its activity to that of wild type SlitOR5. Among other benefits, this approach can provide novel mutants, including constitutively active and null mutants.

If desired, array or microarray-based methods can be used to analyze the expression or the presence of mutation, in SlitOR5 sequences. Array-based methods for minisequencing and for quantitation of nucleic acid expression are well known in the art.

### Transgenic Animals

Transgenic animals expressing SlitOR5 or variants thereof are useful to study the signalling through SlitOR5, as well as for the study of agents that modulate the activity of SlitOR5 and to validate that the agent has an effect on the animal behavior. A transgenic animal is a non-human animal containing at least one foreign gene, called a transgene, which is part of its genetic material. Preferably, the transgene is contained in the animal's germ line such that it can be transmitted to the animal's offspring. A number of techniques may be used to introduce the transgene into an animal's genetic material, including, but not limited to, microinjection of the transgene into pronuclei of fertilized eggs and manipulation of embryonic stem cells. Transgenic animals can carry the transgene in all their cells or can be genetically mosaic. According to the method of conventional transgenesis, additional copies of normal or modified genes are injected into the male pronucleus of the zygote and become integrated into the genomic DNA of the recipient animal. The transgene is transmitted in a Mendelian manner in established transgenic strains. Transgenes can be constitutively expressed or can be tissue specific or even responsive to an exogenous drug, e.g., Tetracycline. A transgenic animal expressing one transgene can be crossed to a second transgenic animal expressing a second transgene such that their offspring will carry and express both transgenes.
Example of such transgenic animals that one can use as tools to study the functional properties of SlitOR5 and to search for modulators is the fly *Drosophila melanogaster.* One can generate UAS-SlitOR5 fly lines that can be further crossed with various Gal4 *Drosophila* lines to target the expression of SlitOR5 in a specific organ (e.g. the antennae) or a specific cell type. Transformed flies can further be screened for electrophysiological or behavioural responses upon stimulation with compound libraries.

### Knock-Out Animals

Animals bearing a homozygous deletion/insertion in the chromosomal sequences encoding SlitOR5 or variants can be used to study the function of the receptor. Of further particular interest is the identification of the specific role of SlitOR5/ Z9,E11-14:OAc in specific physiological and behavioral processes.
For example, one of skill in the art can generate a homozygous SlitOR5 knock-out (e.g. a *Spodoptera*), by creating gene deletions with homologous recombination, or by the use of new technologies such as meganucleases, zing-fingers, TALENs or CRISPR/Cas9.

### Kits

Disclosed herein are kits useful for screening for ligands/agonists/antagonists/inverse agonists of SlitOR5 activity. Kits can include an isolated SlitOR5 polypeptide (including a membrane- or cell-associated SlitOR5 polypeptide, e.g., on isolated membranes, cells expressing SlitOR5, or, on an SPR chip) with or without an isolated Z9,E11-14:OAc. When cells included, said cells may be transformed with a polynucleotide encoding said SlitOR5. Kits may contain a polynucleotide encoding a SlitOR5 polypeptide with or without Z9,E1 1-14:OAc. All kits will comprise means of viewing the activity of said SlitOR5 polypeptide. All kits will comprise the stated items or combinations of items and packaging materials therefore. Kits may also include instructions for use.

### EXAMPLE : Functional characterization of SlitOR5 from Spodoptera littoralis by heterologous expression in Drosophila

### MATERIAL AND METHODS

### Heterologous expression of SlitOR5 in Drosophila

A laboratory strain of *S. littoralis* (Lepidoptera, Noctuidea) was permanently reared on semi-artificial diet at 23°C and 60-70% relative humidity, under a 16:8 light:dark cycle and sexed as pupae. Antennae from 1-day-old male adults were dissected and immediately placed in TRIzol® reagent (Invitrogen, Carlsbad, CA, USA), and total RNA was extracted following manufacturer's instructions. After a DNase I (Promega, Madison, WI, USA) treatment, single stranded cDNA was synthesized from 1 µg of male antennae total RNA using the SuperScript™ II reverse transcriptase (Invitrogen) according to the manufacturer's instructions. The full-length coding sequence of SlitOR5 was then amplified by PCR (Expand High Fidelity PCR System, Roche) using gene-specific primers containing additional restriction sites (forward primer 5'-AACAGATCTGCGGCCGCCAGACGAAGCAGACACCAATTCTA-3', reverse primer 5'-CCTCGAGCCGCGGCCGCACTCATACGTTCATACACTGCAAG-3'). The PCR product was first cloned into pCR®II-TOPO® (Invitrogen), sequenced, and subcloned into the pUAST.attB vector (kindly provided by the Basler lab, University of Zurich, Swiss) using the appropriate restriction sites. Plasmid constructs were purified from liquid cultures of One Shot® TOP10 *E. coli* using the EndoFree Plasmid Maxi kit (Qiagen, Venlo, Netherlands) and sequenced.

The transformant UAS-SlitOR5 balanced fly line was generated by BestGene Inc. (Chino Hills, California, USA), using the PhiC31 integrase system. Briefly, recombinant pUAST.attB-SlitOR5 plasmids were injected into embryos of a *D. melanogaster* line containing an attP insertion site within the second chromosome (genotype y1 M{vas-int.Dm}ZH-2A w*; M{3xP3-RFP.attP'}ZH-51C), leading to non-random integration. To drive expression of SlitOR5 in neurons housed in at1 sensilla, the transformant UAS-SlitOR5 line was crossed to a line carrying the mutant knock-in allele Or67d^{GAL4} (kindly provided by Dr. Barry Dickson, IMP, Vienna, Austria) to generate a double homozygous line w;UAS-SlitOr5;Or67d^{GAL4}. Flies were reared on standard cornmeal-yeast-agar medium and kept in a climate- and light-controlled environment (25°C, 12:12 light:dark cycle). The presence of the UAS-SlitO5 transgene was verified by PCR on genomic DNA extracted from two flies, and the expression of SlitOR5 in fly antennae was verified by RT-PCR on total RNA extracted from ≥100 pairs of antennae.

### Single-sensillum recordings on Drosophila olfactory neurons expressing SlitOR5

The response spectrum of SlitOR5 was tested by means of single-sensillum recordings on flies expressing the SlitOR5 transgene in at1 neurons, instead of the endogenous receptor DmelOR67d. Two- to 10-day-old male flies were restrained in a plastic pipette tip with only the head protruding from the narrow end. The pipette tip was fixed with dental wax on a microscope glass slide with the ventral side of the fly facing upward. Then, the antenna was gently placed on a piece of glass slide and maintained by placing a glass capillary between the 2^{nd} and 3^{rd} antennal segments. Afterwards, the slides were placed under a light microscope (BX51WI, Olympus, Tokyo, Japan) equipped with a 100x magnification objective. Desiccation of the flies was avoided thanks to a constant 1.5 L.min⁻¹ flux of charcoal-filtered and humidified air, delivered through a glass tube of a 7 mm diameter, which terminated ca. 1.5 cm from the antenna.

Stimulation cartridges were built by placing a 1 cm² filter paper in the large opening end of a Pasteur pipette and dropping 10 µl of an odorant solution onto the paper before closing the pipette with a 1 mL plastic pipette tip. A screening panel of 26 pheromone compounds (see Table 1 for detail) were used at a 1 µg/µl dilution in hexane (10 µg deposited on the filter paper). Pipettes with filter papers containing 10 µL of solvent were used as controls. Odorant stimulations were performed by inserting the tip of the pipette into a hole in the glass tube and generating a 500 ms air pulse (0.6 L.min⁻¹), which reached the permanent air flux while going through the stimulation cartridge.

Action potentials were recorded from at1 sensilla using electrolytically sharpened tungsten electrodes. The reference electrode was inserted into the eye of the fly by a manually controlled micromanipulator. The recording electrode was inserted at the base of the sensillum of interest using a motor-controlled PatchStar micromanipulator (Scientifica, Uckfield, UK). The electrical signal was amplifed using a xCellAmp_2c (Dipsi, Chatillon, France), digitized through a Digidata 1440A acquisition board (Molecular Devices, Sunnyvale, CA, USA) then recorded and analyzed using the pCLAMP™ 10 software (Molecular Devices). The net responses of at1 neurons expressing SlitOR5 were calculated by subtracting the spontaneous firing rate (in spikes.s⁻¹) from the firing rate during the odorant stimulation. The time windows used to measure these two firing rates lasted for 500 ms and were respectively placed 500 ms before and 400 ms after the onset of stimulation, to take into account the time for the pheromone compounds to reach the antenna.

The entire pheromone panel was tested 7 times. A pheromone compound was considered as active if the response it elicited was statistically different from the response elicited by the solvent alone (P<0.001 ; Kruskal-Wallis ANOVA followed by a Dunn's *post hoc* test). Then, compounds considered as active were tested in dose-response experiments, conducted with quantities ranging from 100 µg down to the dose necessary to reach the response threshold. Each dilution was tested 5 times.

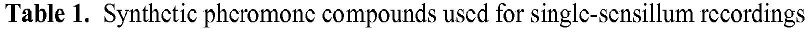

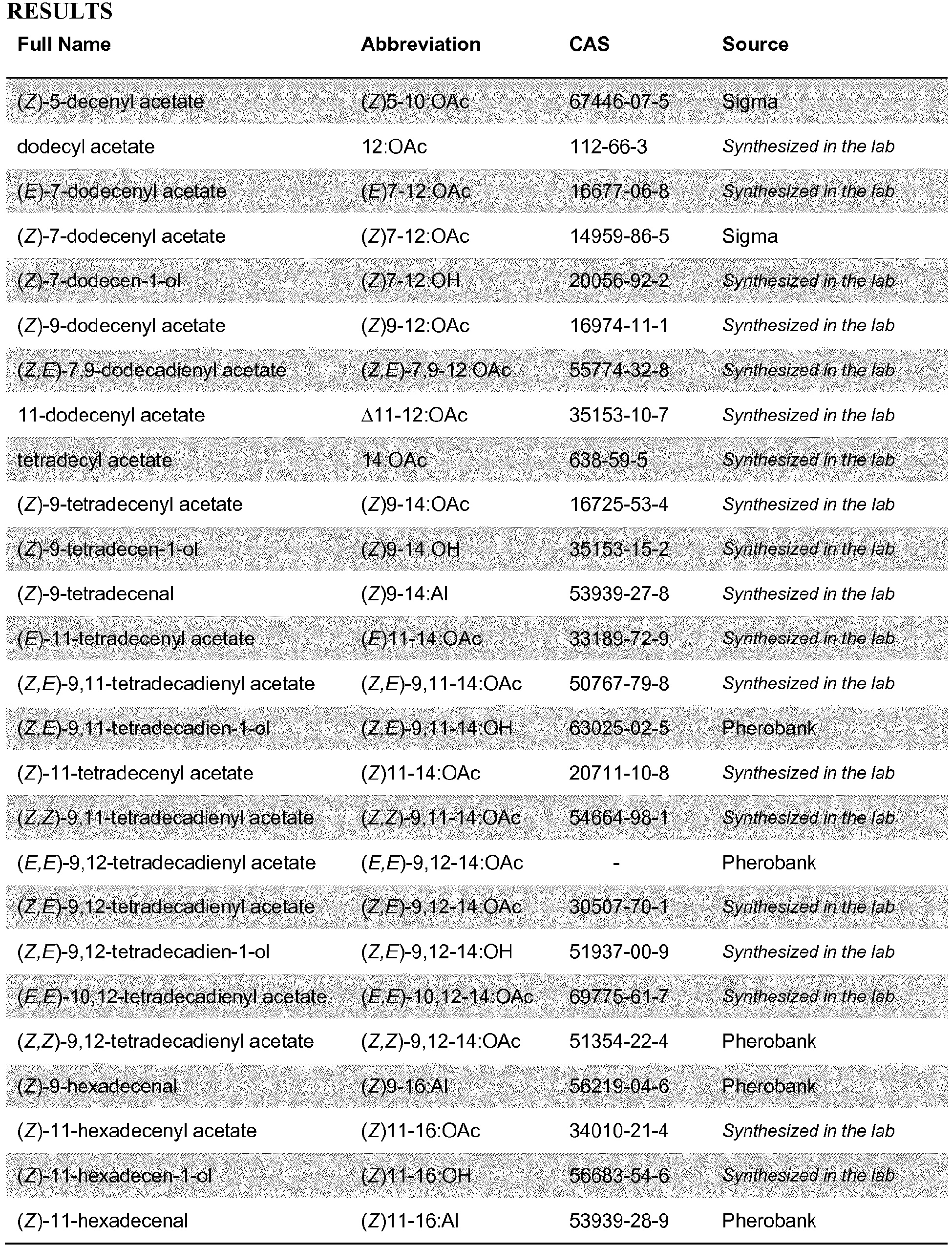

### Response spectrum of SlitOR5-expressing neurons

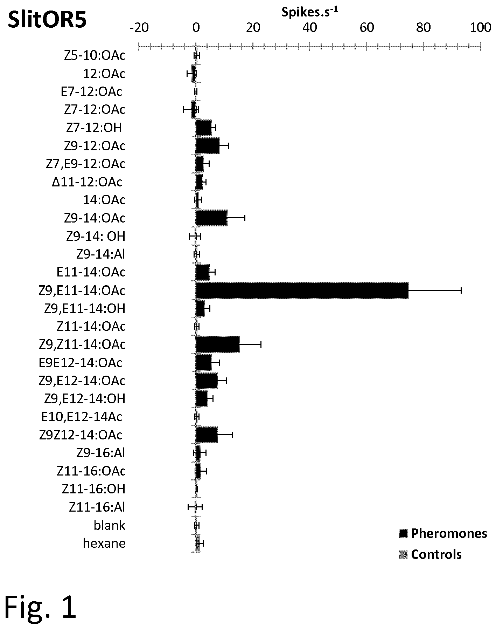

After testing the panel of 26 pheromone compounds 7 times, we observed that only the major pheromone component of *Spodoptera littoralis*, (*ZE*)-9,11-tetradecadienyl acetate (further referred as Z9,E11-14:OAc), elicited a significant response (mean value of 75 spikes.s⁻¹; P<0.01, Kruskall-Wallis ANOVA followed by a Dunn's post-hoc test) in SlitOR5-expressing neurons (Figure 1). Few other pheromone compounds (e.g. Z9-12:OAc, Z9-14:OAc, Z9,Z11-14:OAc) also evoked increases in the firing rate of the neurons when administered, but the responses were not significant compared to the response to the solvent alone.

### Dose-response

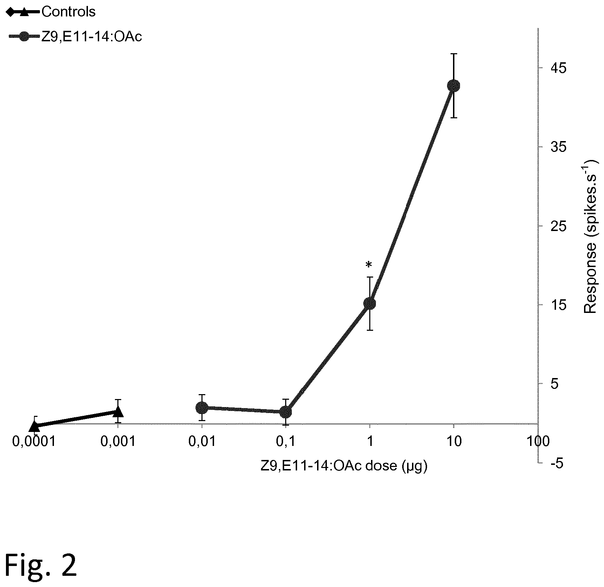

In order to investigate the sensitivity of SlitOR5 towards Z9,E1 1-14:OAc, antennae of SlitOR5-expressing flies were stimulated with increasing doses of the major pheromone compound (0,01 µg to 10 µg loaded on the stimulus cartridge). As presented in Figure 2, significant responses of the at1 neurons were recorded for the doses of 10 µg (43 spikes.s⁻¹; P<0.01; Figure 3A) and 1 µg (15 spikes.s⁻¹; P<0.05; Figure 3B). The responses to the two lower doses did not significantly differ from the mean response to the solvent alone (Figure 3C).

## Claims

1. A method of identifying an antagonist or inverse agonist of the signaling activity of an isolated polypeptide which comprises an amino acid sequence having more than 60%, preferably more than 80%, identity with the amino acid sequence of SlitOR5 from *Spodoptera littoralis* defined in SEQ ID NO:1, comprising :
a) contacting said SlitOR5 polypeptide with Z9,E11-14:OAc in the presence or in the absence of a candidate agent or a sample containing a candidate agent under conditions permitting the binding of said Z9,E1 1-14:OAc to said SlitOR5 polypeptide; and
b) measuring the binding of said SlitOR5 polypeptide to said Z9,E11-14:OAc or a signaling activity of said SlitOR5 polypeptide,
c) comparing the binding or the signaling activity in the presence of said candidate agent or sample with the binding, respectively the signaling activity, in the absence of said agent or sample.

2. The method according to claim 1, wherein said isolated polypeptide comprises the amino acid sequence defined in SEQ ID NO:1.

3. A method of identifying an agonist of the signaling activity of an isolated polypeptide which comprises an amino acid sequence having more than 60%, preferably more than 80%, identity with the amino acid sequence of SlitOR5 from *Spodoptera littoralis* defined in SEQ ID NO:1, said method comprising:
a) contacting said SlitOR5 polypeptide with a candidate agent or a sample containing a candidate agent;
b) measuring a signaling activity of said SlitOR5 polypeptide in the presence of said agent or sample; and
c) comparing said activity measured in the presence of said agent or sample to said activity measured in a reaction in which said SlitOR5 polypeptide is contacted with Z9,E11-14:OAc.

4. The method according to claim 3, wherein said isolated polypeptide comprises the amino acid sequence defined in SEQ ID NO:1.

## Patentansprüche

1. Verfahren zum Identifizieren eines Antagonisten oder inversen Agonisten der Signalaktivität eines isolierten Polypeptids, das eine Aminosäuresequenz umfasst, die mehr als 60 %, vorzugsweise mehr als 80 % Identität mit der in SEQ ID-Nr.: 1 definierten Aminosäuresequenz von SlitOR5 von *Spodoptera littoralis* aufweist, umfassend:
a) Inkontaktbringen des SlitOR5-Polypeptids mit Z9, E11-14:OAc in Gegenwart oder in Abwesenheit eines Kandidatenmittels oder einer Probe, die ein Kandidatenmittel enthält, unter Bedingungen, die die Bindung des Z9, E11-14:OAc an das SlitOR5-Polypeptid ermöglichen; und
b) Messen der Bindung des SlitOR5-Polypeptids an das Z9,E11-14:OAc, oder einer Signalaktivität des SlitOR5-Polypeptids,
c) Vergleichen der Bindung oder der Signalaktivität in Gegenwart des Kandidatenmittels oder der Probe mit der Bindung bzw. der Signalaktivität in Abwesenheit des Mittels oder der Probe.

2. Verfahren nach Anspruch 1, wobei das isolierte Polypeptid die in SEQ ID-Nr.: 1 definierte Aminosäuresequenz umfasst.

3. Verfahren zum Identifizieren eines Agonisten der Signalaktivität eines isolierten Polypeptids, das eine Aminosäuresequenz umfasst, die mehr als 60 %, vorzugsweise mehr als 80 % Identität mit der in SEQ ID-Nr.: 1 definierten Aminosäuresequenz von SlitOR5 von *Spodoptera littoralis* aufweist, wobei das Verfahren umfasst:
a) Inkontaktbringen des SlitOR5-Polypeptids mit einem Kandidatenmittel oder einer Probe, die ein Kandidatenmittel enthält;
b) Messen einer Signalaktivität des SlitOR5-Polypeptids in Gegenwart des Mittels oder der Probe; und
c) Vergleichen der in Gegenwart des Mittels oder der Probe gemessenen Aktivität mit der Aktivität, die in einer Reaktion gemessen wird, in der das SlitOR5-Polypeptid mit Z9, E11-14:OAc in Kontakt gebracht wird.

4. Verfahren nach Anspruch 3, wobei das isolierte Polypeptid die in SEQ ID-Nr.: 1 definierte Aminosäuresequenz umfasst.

## Revendications

1. Méthode d'identification d'un antagoniste ou d'un agoniste inverse de l'activité de signalisation d'un polypeptide isolé qui comprend une séquence d'acides aminés présentant une identité supérieure à 60 %, de préférence supérieure à 80 %, avec la séquence d'acides aminés de SlitOR5 de *Spodoptera littoralis* définie dans SEQ ID NO : 1, comprenant :
a) la mise en contact dudit polypeptide SlitOR5 avec Z9, E11-14:OAc en présence ou en l'absence d'un agent candidat ou d'un échantillon contenant un agent candidat dans des conditions permettant la liaison dudit Z9, E11-14:OAc audit polypeptide SlitOR5 ; et
b) la mesure de la liaison dudit polypeptide SlitOR5 audit Z9, E11-14:OAc ou d'une activité de signalisation dudit polypeptide SlitOR5,
c) la comparaison de la liaison ou de l'activité de signalisation en présence dudit agent candidat ou échantillon avec la liaison, respectivement l'activité de signalisation, en l'absence dudit agent ou échantillon.

2. Méthode selon la revendication 1, dans laquelle ledit polypeptide isolé comprend la séquence d'acides aminés définie dans SEQ ID NO : 1.

3. Méthode d'identification d'un agoniste de l'activité de signalisation d'un polypeptide isolé qui comprend une séquence d'acides aminés présentant une identité supérieure à 60 %, de préférence supérieure à 80 %, avec la séquence d'acides aminés de SlitOR5 de *Spodoptera littoralis* définie dans SEQ ID NO : 1, ladite méthode comprenant :
a) la mise en contact dudit polypeptide SlitOR5 avec un agent candidat ou un échantillon contenant un agent candidat ;
b) la mesure de l'activité de signalisation dudit polypeptide SlitOR5 en présence dudit agent ou échantillon ; et
c) la comparaison de ladite activité mesurée en présence dudit agent ou échantillon à ladite activité mesurée dans une réaction dans laquelle ledit polypeptide SlitOR5 est mis en contact avec Z9,E11-14:OAc.

4. Méthode selon la revendication 3, dans laquelle ledit polypeptide isolé comprend la séquence d'acides aminés définie dans SEQ ID NO : 1.
